# EUROPEAN PATENT APPLICATION

(11) **EP 4 446 386 A1**
(43) Date of publication of application: **16.10.2024**
(21) Application number: 23168138.8
(22) Date of filing: 15.04.2023
(51) Int. Cl.: C09B 23/14, C07D 209/10, C07D 209/12, C07D 209/14

(54) **3,3-DIMETHYL-2-STYRYL-3H-INDOLE DERIVATIVES AND METHOD FOR THE PRODUCTION THEREOF**

(71) Applicant: Kaunas University of Technology, 44029 Kaunas (LT)
(72) Inventor: Sackus, Algirdas, Kaunas (LT); Arbaciauskiene, Egle, Kaunas (LT); Varvuolyte, Gabriele, Kaunas (LT); Krikstolaityte, Sonata, Kaunas (LT); Kleiziene, Neringa, Kaunas (LT); Bieliauskas, Aurimas, Kaunas (LT); Martynaitis, Vytas, Kaunas (LT); Raskauskiene, Greta, Kaunas (LT); Gudelis, Emilis, Kaunas (LT)
(74) Representative: Klimaitiene, Otilija

(57) **Abstract**

The invention describes new 3,3-dimethyl-2-styryl-3H-indole derivatives and their synthesis method using a flow reactor. The compounds of the invention are obtained in 61-98% yield by condensation of the corresponding 3H-indole chlorides with corresponding benzaldehydes for 1 hour in ethylene glycol at 125 °C. The stream is then cooled and passed through a column filled with a sorbent that binds the unreacted aldehyde. After evaporation of the solvent, it is returned to the preparation of the initial solutions, and the residue is purified by chromatography. New derivatives of 3,3-dimethyl-2-styryl-3H-indole can be used as pH sensors or intermediates for the synthesis of other special dyes (pH sensors of biological media, cell markers, etc.).

## Description

### FIELD OF THE INVENTION

The invention is attributed to the new 3,3-dimethyl-2-styryl-3*H*-indole derivatives and the method of their production by the flow synthesis. The new 3,3-dimethyl-2-styryl-3*H*-indole derivatives can be used in chemical industry, medical diagnostics, etc., and the proposed production method is characterized by high reaction efficiency and low cost.

### BACKGROUND OF THE INVENTION

The 3,3-dimethyl-2-styryl-3*H*-indole derivatives are relevant products used in the chemical industry, scientific research, as well as in the study of processes in living cells and medical diagnostics. For example, (*E*)-3,3-dimethyl-2-styryl-3*H*-indoles have been synthesized as analogs of vinca alkaloids and tested for their anticancer activity. The compounds showed moderate antitumor activity against MCF-7, MDA-MB-231, HepG2, HepG2/ADM and K562 cells [CN102942515A; Z. Jing, D. Lijuan, C. Minfeng, X. Xuzhi, X. Shengwei, G. Cuiping, X. Gaokeng, B. Liangliang, Y. Wencai, Z. Dongmei et al. Elaboration of thorough simplified vinca alkaloids as antimitotic agents based on pharmacophore similarity. European Journal of Medicinal Chemistry. 2011, 65, 158-167]. The *(*E)-3,3-dimethyl-2-styryl-3*H*-indoles have also been used as charge carriers in electrophotography [JP54110834A, DE2054253B2] and also as building blocks for the synthesis of dye materials. A strategy for the synthesis of indolino-azepines is described, when 2-styryl-3*H*-indoles react with spiro[2-cyclopropene-1,9'-fluorenes] to form betaines, which in the 1,7-electrocyclization reaction form indolino-azepines with reversible halochromic properties. These compounds are used as acid- or UV-activated copying agents [Y. Ma, C. Weber, T. Hartmann, H. Durr, C. Kruger, J. Kossanyi. A Simple Access to Indolino-Azepines - A New Halochromic System for Information Recording. Synthesis, 2001, 12, 1812-1817]. Also, Mushkalo *et al.* described the synthesis of colored norcyanines from 2-styryl-3*H*-indoles [L. K. Mushkalo, M. Habubi, N. N. Mushkalo, L. V. Fedorova. Norcyanines in the indolenine series. Ukrainskii Khimicheskii Zhurnal (Russian Edition). 1974, 40(9), 957-962].

Some derivatives containing the indole fragment, usually complex conjugated systems, exhibit sensitivity to changes in the pH of the medium and are known as pH sensors [Y. Liu, Y. Zhang, J. Wang, A. Yang, Y. Zhao, A. Zhou, R. Xiong, C. Huang. Bioorganic Chemistry. 2022; 124: 10572; Y. Zhang, Y. Zhao, B. Song, C. Huang. Dyes and Pigments. 2021; 188: 109205; L. Fan, S.-Q. Gao, Z-B Li, W.-F. Niu, W.-J. Zhang, S.-M. Shuang, C. Dong. Sensors and Actuators B. 2015; 221, 1069-1076.]

The closest prior art document [Jing Zheng, Lijuan Deng, Minfeng Chen, Xuzhi Xiao, Shengwei Xiao, Cuiping Guo, Gaokeng Xiao, Liangliang Bai, Wencai Ye, Dongmei Zhang, Heru Chen, Elaboration of thorough simplified vinca alkaloids as antimitotic agents based on pharmacophore similarity, European Journal of Medicinal Chemistry (2013), 65, 158-167] discloses a synthesis of 3,3-dimethyl-2-styryl-3*H*-indole derivatives from 2,3,3-trimethyl-3*H*-indole salt and aromatic carbaldehyde. The starting materials are mixed in ethanol at boiling temperature in a flask for 4 h. Products are obtained in 60-90% yields. Known method of synthesis of 3,3-dimethyl-2-styryl-3*H*-indole derivatives using batch method is not efficient enough: in order to obtain compounds in larger quantities, it is more difficult to control the stoichiometry of the reaction, i.e. to change the concentration of one reactant in relation to another. Also, insufficient stability of temperature control fails to reduce degradation and by-product formation; overheating zones can appear, the time of obtaining products is too long and the energy efficiency is too low for the needs of modern industry; conditions are not created for a closed technological cycle and its repetition. The present invention seeks to provide a flow chemistry process for producing 3,3-dimethyl-2-styryl-3*H*-indole derivatives and to address some of the shortcomings of the prior art.

### SUMMARY OF THE INVENTION

In order to solve the above-mentioned problems and at the same time to expand the variety of compounds sensitive to changes in the pH of the medium by adding another simple structure, new derivatives of 3,3-dimethyl-2-styryl-3*H*-indole are proposed and a method for their preparation is developed, of which all the features are described in the claims of the invention.

The subject of the invention are new 3,3-dimethyl-2-styryl-3*H*-indole derivatives with the general formula (I) 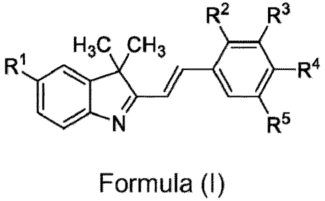 wherein
- R¹: represents hydrogen, Cl or methyl;
- R²: represents hydrogen or methoxy;
- R³: represents hydrogen;
- R³: represents trifluoromethyl when R⁵ represents trifluoromethyl; or
- R² and R³: together are a chain from -CH=CH-CH=CH-;
- R⁴: represents hydrogen, piperidin-1-yl-, pyrrolidin-1-yl-;
- R⁴: represents methoxy when R² represents methoxy;
- R⁴: represents dimethylamino, diethylamino, when R¹ does not represent a hydrogen;
- R⁵: represents hydrogen.

More specifically, the 3,3-dimethyl-2-styryl-3*H*-indole derivative is selected from the following compounds:
(*E*)-3,3-dimethyl-2-(2,4-dimethoxystyryl)-3*H*-indole (**Ia**);
(*E*)-3,3,5-trimethyl-2-(4-dimethylaminostyryl)-3*H*-indole (**Ib**);
(*E*)-3,3-dimethyl-2-[3,5-bis(trifluoromethyl)styryl]-3*H*-indole **(Ic);**
(*E*)-5-chloro-3,3-dimethyl-2-(4-dimethylaminostyryl)-3*H*-indole (**Id**);
(*E*)-2-(4-diethylaminostyryl)-3,3,5-trimethyl-3*H*-indole (**Ie**);
(*E*)-3,3-dimethyl-2-[4-(piperidin-1-yl)styryl]-3*H*-indole (**If**);
(*E*)-3,3-dimethyl-2-[4-(pyrrolidin-1-yl)styryl]-3*H*-indole (**Ig**); or
(*E*)-3,3-dimethyl-2-[2-(naphthalen-1-yl)ethen-1-yl]-3*H*-indole (**Ih**).

It turned out that the specified 3,3-dimethyl-2-styryl-3*H*-indole derivatives have the property of changing color depending on the pH of the medium. They can be used as indicators of pH change in the range from 1.3÷1.7 to 5.9÷6.3.

The object of the present invention is a new method for the production of 3,3-dimethyl-2-styryl-3H-indole derivatives according to the following formula (I) 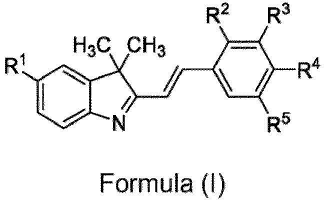 wherein
R¹ represents hydrogen, halogen, alkyl or aryl radical;
R² represents hydrogen or methoxy;
R³ represents hydrogen, methoxy or trifluoromethoxy; or
R² and R³ together are a chain from -CH=CH-CH=CH-;
R⁴ represents a hydroxy, methoxy, dimethylamino, diethylamino, piperidin-1-yl-, pyrrolidin-1-yl- or morpholin-4-yl- radical;
R⁵ represents hydrogen or trifluoromethoxy radical.

The production method comprises condensation of the 2,3,3-trimethyl-3*H*-indole salt with the corresponding aromatic aldehyde in ethylene glycol.

The new method is characterized in that the reaction is carried out in a flow reactor and the method comprises the following steps:
a) preparing solutions of the initial compounds of 2,3,3-trimethyl-3*H*-indole salt (A) and the corresponding aromatic aldehyde (B) in ethylene glycol in separate reservoirs;
b) feeding the prepared solutions (A) and (B) into the T-shaped mixer maintaining a constant flow;
c) after mixing the reaction mixture, moving at the given speed, is heated in the tubular zone of the flow reactor for 50-70 min, optimally 60 min, at 120-130 °C, optimally at 125 °C;
d) cooling the continuous flow of the solution containing the reaction product in the reactor loop with ambient air to about 30 °C;
e) carrying out separation of unreacted aldehyde;
f) followed by separation of the ethylene glycol;
g) the isolated reaction product is purified by a chromatographic column.

In one of the most optimal alternatives of the method
- before the stage a), the flow reactor is filled up with solvent ethylene glycol and heated to the reaction temperature;
   a) stage: the initial compounds are 2,3,3-trimethyl-3*H*-indole hydrochloride (A) and the correspondingly substituted benzaldehyde (B);
   b) stage: solutions A and B are prepared and supplied independently but simultaneously in a molar ratio of 1:1.1, mixed in a T-mixer and fed to the reactor at a given flow rate, for example 0.266 mL/min
   d) stage: the reaction mixture is cooled with air to a temperature of 30 °C;
   e) stage: to separate the unreacted aldehyde, the reaction flow is passed through a chromatographic column with an isoniazid-modified sorbent with a sorption capacity of 0.11-0.28 mmol/g for aldehyde;
   f) stage: the separation of the solvent is carried out by evaporating the ethylene glycol in a vacuum evaporator and returning the separated ethylene glycol to the stage a) for the preparation of the initial solutions.

### SHORT DESCRIPTION OF DRAWINGS

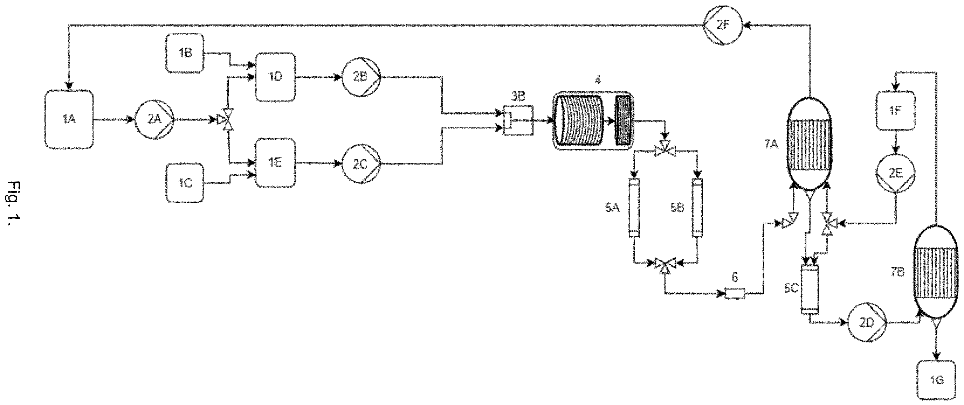

The general technological scheme of the proposed synthesis method is shown in Fig. 1, in which:
1 A-G Material containers; 2 A-F Pumps; 3B Mixer; 4 Flow reactor; 5 A-C Purification columns; 6 Back pressure regulator; 7 A-B Evaporators.

### DETAILED DESCRIPTION OF THE INVENTION

A flow condensation system of 3*H*-indole salts and aromatic aldehydes was developed for the synthesis of 3,3-dimethyl-2-styryl-3*H*-indole derivatives, the reaction being carried out in a flow reactor, using ethylene glycol as a solvent.

The general reaction scheme for the synthesis of the compounds, i.e. 2-styryl-3,3-dimethyl-3*H*-indoles by the invention can be represented as follows:

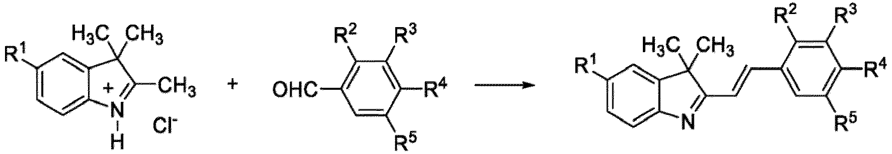

### Description of the general technological scheme shown in Fig. 1

2,3,3-Trimethyl-3*H*-indole salt (A) from the container 1B and the corresponding aldehyde (B) from the container 1C are dissolved in ethylene glycol in separate reservoirs 1D and 1E (Solution A and solution B). The solutions prepared from the reservoirs are dosed and mixed by the pumps 2B and 2C in the T-mixer 3B, and the solution flow of the initial compounds enters the tubular zone 4 of the flow reactor. Reagents A and B may be introduced together into the mixing device 3B so that a desired molar ratio of reagents A, B, for example 1:1.1, is mixed prior to introduction into the flow reactor. In the heated tubular zone 4, the reaction mixture is maintained at 125 °C for an appropriate period of time, maintaining the flow rate, and in the cooled tubular zone, it is air-cooled to ambient temperature.

The reaction mixture leaving the flow reactor is fed to a column with an isoniazid-modified sorbent (5A or 5B), which chemically sorbs the unreacted aldehyde.

The flow that left the column is directed by pump 6 to distillation unit 7A, where ethylene glycol is distilled. Distilled ethylene glycol is returned to tank 1A by pump 2F for the preparation of initial material solutions. The residue obtained after distillation is dissolved in an eluent and eluted in a chromatographic column 5C. After collecting the appropriate fraction, the pure product solution is pumped by the pump 2D into the evaporator 7B, and after evaporating the eluent, the target product is obtained in the container 1G. The evaporated eluent, modified in the container 1F, is returned to the evaporator 7A by the pump 2E to prepare the chromatographic solution, and is also supplied to the chromatography column 5C.

### EMBODIMENTS OF THE INVENTION

The following is information on specific examples describing how the compounds of the invention were obtained. Embodiments of the invention are provided for illustration, they do not limit the scope of the invention.

### Example 1

### General description of the synthesis of the compounds of the invention using a flow reactor

A flow reactor with a capacity of 16 mL was used for the synthesis. The synthesis was carried out according to the technological scheme described above.

Preparation of the reactor. The reactor loop is charged with 16 mL of ethylene glycol and heated to 125 °C.

Preparation of the stock solutions. The corresponding initial compounds, i.e. 3H-indole hydrochloride (1 mole, 1 equiv.) and aromatic aldehyde (1.1 equiv.) were dissolved in ethylene glycol (5 mL each) in separate vessels. Prepared solution A (0.2 M) and solution B (0.22 M) were pumped into the T-mixer at a flow rate of 0.133 mL/min. The flows remained constant throughout the reaction.

Execution of the reaction. The reaction mixture was fed into the reactor at a flow rate of 0.266 mL/min. The reaction was carried out in the reactor loop at a temperature of 125 °C and a retention time of 60 min at atmospheric pressure. The continuous flow was cooled to about 30 °C (by cooling in the reactor cooling loop with ambient air).

To separate the unreacted aldehyde, the cooled stream was passed through the isoniazid-modified sorbent at a flow rate of 0.266 mL/min through the isoniazid-modified sorbent Amberlyst-ISO column; sorbent sorption capacity for aldehyde 0.11-0.28 mmol/g. When the flow reaction was carried out for about one hour, the required amount of sorbent was 12.1g.

Regeneration of the solvent ethylene glycol from the reaction mixture was carried out in the vacuum distiller at a temperature of 120 °C and a pressure of 0.001 bar. The separated ethylene glycol was returned to the preparation of initial solutions.

The product was purified by column chromatography (stationary phase - silica gel, eluent hexane/ethyl acetate).

Process performance was 2.208-3.136 mmol/h 729-967 mg/h.

Compounds **Ia-Ih** were synthesized by the described flow synthesis method with the following substituents:
**Ia**: R¹, R³, R⁵ = H; R², R⁴ = CHsO;
**Ib**: R¹ = CH₃; R², R³, R⁵ = H; R⁴ = N(CH₃)₂;
**Ic**: R¹, R², R⁴ = H; R³, R⁵ = CF₃;
**Id**: R¹ = Cl; R², R³, R⁵ = H; R⁴ = N(CH₃)₂;
**Ie**: R¹ = CH₃; R², R³, R⁵ = H; R⁴ = N(CH₂CH₃)₂;
**If**: R¹, R², R³, R⁵ = H; R⁴ = piperidin-1-yl;
**Ig**: R¹, R², R³, R⁵ = H; R⁴ = pyrrolidin-1-yl;
**Ih**: R¹, R⁴, R⁵ = H; R² and R³ = -CH=CH-CH=CH-;

### Example 2

### (E)-3,3-dimethyl-2-(2,4-dimethoxystyryl)-3H-indole (Ia);

Carrying out the reaction according to the general description of the flow synthesis process (Example 1) from 2,3,3-trimethyl-3*H*-indole hydrochloride (195 mg, 1 mmol) and 2,4-dimethoxybenzaldehyde (183 mg, 1.1 mmol) gave 233 mg (76%) of compound **Ia**. An orange colored amorphous material was obtained.

¹H NMR (700 MHz, CDCl₃): δ 1.47 (s, 6H, 2×3-CH₃), 3.85 (s, 3H, OCH₃), 3.90 (s, 3H, OCH₃), 6.48 (d, *J* = 2,1 Hz, 1H, 3-Ph), 6.55 (dd, *J* = 8.4 Hz, *J* = 2.1 Hz, 1H, 5-Ph), 7.07 (d, *J =* 16.8 Hz, 1H, CH=CH), 7.21 (dt, *J* = 7.0 Hz, *J =* 0.7 Hz, 1H, 5-H), 7.31 (d, *J* = 7.0 Hz, 1H, 7-H), 7.32 (dt, *J* = 7.0 Hz, *J* = 0.7 Hz, 1H, 6-H), 7.58 (d, *J* = 8.4 Hz, 1H, 6-Ph), 7.62 (d, *J* = 7.0 Hz, 1H, 4-H), 7.94 (d, ³*J* = 16.8 Hz, 1H, CH=CH). ¹³C NMR (175 MHz, CDCl₃): δ 24.4, 22.7, 55.6, 55.7, 98.6, 105.4, 118.4, 118.6, 120.5, 121.1, 125.3, 127.8, 129.0, 133.3, 146.9, 154.3, 159.4, 162.1, 184.4. FT-IR (cm⁻¹): 3002, 3057 C-H_{arom.}; 2836, 2864, 2928, 2961 C-H_{alif.}; 1602 (CH=CH). HRMS (ESI) m/z [M + H]⁺ calcd C₂₀H₂₂NO₂ 308.1651; found 308.1645.

The synthesis of compound **Ia** was also attempted in a flask. Initial compounds, i.e. 3H-indole hydrochloride (1 mmol, 1 equiv) and 2,4-dimethoxybenzaldehyde (1.3 equiv) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:1, v/v). The yield of product **Ia** was 65%.

Unlike synthesis in a flask, the product was obtained in a better yield in a flow reactor. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 3

### (E)-3,3,5-trimethyl-2-(4-dimethylaminostyryl)-3H-indole (Ib)

Carrying out the reaction according to the general description of the flow synthesis process, from 2,3,3,5-tetramethyl-3*H*-indole hydrochloride (209 mg, 1 mmol) and 4-(dimethylamino)benzaldehyde (164 mg, 1.1 mmol), 283 mg (93%) of compound **Ib** was obtained. A yellow-orange amorphous material was obtained.

¹H NMR (700 MHz, CDCl₃): δ 1.44 (s, 6H, 2×3-CH₃), 2.42 (s, 3H, 5-CH₃), 3.02 (s, 6H, N(CH₃)₂), 6.70-6.72 (m, 2H, 3,5-Ph), 6.86 (d, ³*J* = 16.8 Hz, 1H, CH=CH), 7.11 (s, 1H, 4-H_{Ind}), 7.12 (d, *J* = 7.7 Hz, 1H, 7-H_{Ind}). 7.48 (d, *J* = 7.7 Hz, 1H, 6-H_{Ind}), 7.50-7.51 (m, 2H, 2,6-Ph), 7.64 (d, ³*J* = 16.8 Hz, 1H, CH=CH). ¹³C NMR (175 MHz, CDCl₃): δ 21.7, 24.4, 40.4, 52.4, 112.2, 115.3, 119.8, 121.9, 124.3, 128.4, 129.0, 134.9, 138.1, 146.8, 151.2, 152.2, 183.3. FT-IR (cm⁻¹): 3003, 3029, 3080 C-H_{arom.}; 2808, 2861, 2924, 2966 C-H_{alif.}; 1595 (CH=CH). HRMS (ESI) m/z [M + H]⁺ calcd C₂₁H₂₅N₂ 305.2018; found 305.2012.

The synthesis of compound **Ib** was also attempted in a flask. Initial compounds, i.e. 3*H-*indole hydrochloride (1 mmol, 1 equiv) and 4-(dimethylamino)benzaldehyde (1.3 equiv) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:1, v/v). The yield of product **I**b was 81%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 4

### (E)-3,3-dimethyl-2-[3,5-bis(trifluoromethyl)styryl]-3H-indole (Ic)

Reaction according to the general flow synthesis description gave 199 mg (52%) of compound **Ic** from 2,3,3-trimethyl-3*H*-indole hydrochloride (195 mg, 1 mmol) and 3,5-bis(trifluoromethyl)benzaldehyde (0.18 mL, 1.1 mmol). A brownish amorphous material was obtained.

¹H NMR (700 MHz, CDCl₃): δ 1.49 (s, 6H), 7.19 (d, *J* = 16.3 Hz, 1H), 7.28 (dd, *J* = 16.7, 9.1 Hz, 1H), 7.32 - 7.40 (m, 2H), 7.67 (d, *J* = 7.7 Hz, 1H), 7.75 (d, *J* = 16.3 Hz, 1H), 7.83 (s, 1H), 8.01 (s, 2H). ¹³C NMR (175 MHz, CDCl₃): δ 23.6, 52.8, 121.2, 122.3 (hept, ³*J*_{C,F} = 3.9 Hz), 123.2 (q, ¹*J*_{C,F} = 272.8 Hz), 123.5, 126.4, 127.0 (q, ³*J*_{C,F} = 3.8 Hz), 128.1, 132.4 (q, ²*J*_{C,F} = 33.4 Hz), 134.2, 138.2, 146.6, 153.5, 182.1. δ FT-IR (cm⁻¹): 2968, 2932, 2909, 2868 C-Hₐᵣₒₘ, C-H_{alif}, 1381, 1276, 1171, 1126, 968, 893, 845, 758, 681. HRMS (ESI) m/z [M + H]⁺ calcd C₂₀H₁₆F₆N 384.1181; found 384.1184.

The synthesis of compound **Ic** was also attempted in a flask. Initial 3*H*-indole hydrochloride (1 mmol, 1 equiv.) and 3,5-bis(trifluoromethyl)benzaldehyde (1.3 equiv.) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:1, v/v). The yield of product **Ic** was 15%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 5

### (E)-5-chloro-3,3-dimethyl-2-(4-dimethylaminostyryl)-3H-indole (Id)

Reaction according to the general flow synthesis description gave 301 mg (93%) of compound **Id** from 5-chloro-2,3,3-trimethyl-3*H*-indole hydrochloride (231 mg, 1 mmol) and 4-(dimethylamino)benzaldehyde (164 mg, 1.1 mmol). A red/brown amorphous material was obtained.

¹H NMR (700 MHz, CDCl₃): δ 1.44 (s, 6H, 2×3-CH₃), 3.02 (s, 6H, N(CH₃)₂), 6.70-6.71 (m, 2H, 3,5-Ph), 6.82 (d, ³*J* = 16.1 Hz, 1H, CH=CH), 7.25 (d, *J* = 2.1 Hz, 1H, 4-H), 7.28 (dd, *J* = 8.4 Hz, *J* = 2.1 Hz, 1H, 6-H), 7.49 (d, *J* = 8.4 Hz, 1H, 7-H), 7.50 (m, 2H, 2,6-Ph), 7.68 (d, *J=* 16.1 Hz, 1H, CH=CH). ¹³C BMR (175 MHz, CDCl₃): δ 24.1, 40.3, 53.0, 112.2, 114.4, 120.9, 121.8, 123.9, 128.0, 129.3, 130.6, 139.2, 148.3, 151.4, 153.0, 184.6. FT-IR (cm⁻¹): 3036, 3079 C-H_{arom.}; 2802, 2866, 2900, 2925, 2970 C-H_{alif.}; 1594 (CH=CH); 809 C-Cl. HRMS (ESI) m/z [M + H]⁺ calculated C₂₀H₂₂CIN₂ 325.1472; found 325.1466.

The synthesis of compound **Id** was also attempted in a flask. Initial 3*H*-indole hydrochloride (1 mmol, 1 equiv) and 4-(dimethylamino)benzaldehyde (1.3 equiv.) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:1, v/v). The yield of product **Id** was 80%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 6

### (E)-2-(4-diethylaminostyryl)-3,3,5-trimethyl-3H-indole (le)

Reaction according to the general flow synthesis description gave 315 mg (95%) of compound **Ie** from 2,3,3,5-tetramethyl-3*H*-indole hydrochloride (209 mg, 1 mmol) and 4-(diethylamino)benzaldehyde (195 mg, 1.1 mmol). A red colored amorphous material was obtained.

¹H NMR (700 MHz, CDCl₃): δ 1.20 (t, ³*J* = 7.0 Hz, 6H, 2×CH₂CH₃), 1.44 (s, 6H, 2×3-CH₃), 2.41 (s, 3H, 5-CH₃), 3.40 (q, ³*J* = 7.0 Hz, 4H, 2×CH₂CH₃), 6.66-6.67 (m, 2H, 3,5-Ph), 6.83 (d, ³*J* = 16.4 Hz, 1H, CH=CH), 7.10 (s, 1H, 4-H_{Ind}), 7.12 (d, *J* = 7.7 Hz, 1H, 7-H_{Ind}), 7.47 (d, *J=* 7.7 Hz, 1H, 6-H_{Ind}), 7.47-7,48 (m, 2H, 2,6-Ph), 7.63 (d, ³*J* = 16.4 Hz, 1H, CH=CH). ¹³C NMR (175 MHz, CDCI₃): δ 12.8, 21.7, 24.3, 44.6, 52.4, 111.6, 114.66, 119.69, 121.9, 123.4, 128.4, 129.3, 134.8, 138.2, 146.8, 148.7, 152.2, 183.4. FT-IR (cm⁻¹): 3001, 3034 C-H_{arom.}; 2867, 2925, 2967 C-H_{alif.}; 1593 (CH=CH). HRMS (ESI) m/z [M + H]⁺ calculated C₂₃H₂₉N₂ 333.2331; found 333.2325.

The synthesis of compound **Ie** was also attempted in a flask. Initial 3*H*-indole hydrochloride (1 mmol, 1 equiv.) and 4-(diethylamino)benzaldehyde (1.3 equiv.) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:1, v/v). The yield of product **Ie** was 52%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 7

### (E)-3,3-dimethyl-2-[4-(piperidin-1-yl)styryl]-3H-indole (If);

Reaction according to the general flow synthesis description gave 228 mg (69%) of compound **If** from 2,3,3-trimethyl-3*H*-indole hydrochloride (195 mg, 1 mmol) and 4-(1-piperidinyl)benzaldehyde (208 mg, 1.1 mmol). A red/brown amorphous material was obtained.

¹H NMR (700 MHz, CDCl₃): δ 1.45 (s, 6H, 2×CH₃), 1.60-1.72 (m, 6H, 3×CH₂ₚᵢₚₑᵣ), 3.27-3.28 (m, 4H, 2×CH₂ₚᵢₚₑᵣ), 6.90 (d, *J* = 16.1 Hz, 1H, CH=CH), 6.90-6.91 (m, 2H, 3,5-Ph), 7.20 (t, *J* = 7.0 Hz, 1H, 5-H_{Ind}), 7.30 (d, *J* = 7.0 Hz, 1H, 7-H_{Ind}), 7.32 (t, *J* = 7.7 Hz, 1H, 6-H_{Ind}), 7.50-7.51 (m, 2H, 2,6-Ph), 7.60 (d, *J* = 7.7 Hz, 1H, 4-H_{Ind}), 7.67 (d, ³*J* = 16.1 Hz, 1H, CH=CH). ¹³C NMR (175 MHz, CDCl₃): δ 24.2, 24.5, 25.6, 49.6, 52.6, 115.4, 116.0, 120.3, 121.1, 125.2, 126.1, 127.9, 129.0, 138.3, 146.6, 152.6, 154.3, 184.0. FT-IR (cm⁻¹): 3007, 3035 C-H_{arom.}; 2811, 2854, 2928, 2960 C-H_{alif.}; 1597 (CH=CH). HRMS (ESI) m/z [M + H]⁺ calculated C₂₃H₂₇N₂ 331.2174; found 331.2169.

The synthesis of compound **If** was also attempted in a flask. Initial 3*H*-indole hydrochloride (1 mmol, 1 equiv) and 4-(1-piperidinyl)benzaldehyde (1.3 equiv) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:2, v/v). The yield of product **Ie** was 55%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 8

### (E)-3,3-dimethyl-2-[4-(pyrrolidin-1-yl)styryl]-3H-indole (Ig);

Reaction according to the general flow synthesis description gave 268 mg (85%) of compound **Ig** from 2,3,3-trimethyl-3*H*-indole hydrochloride (195 mg, 1 mmol) and 4-(1-pyrrolidinyl)benzaldehyde (193 mg, 1.1 mmol). A red/brown colored amorphous material was obtained.

¹H NMR (700 MHz, CDCl₃): δ 1.46 (s, 6H, 2×CH₃), 2.01-2.03 (m, 4H, 2×NCH₂CH₂), 3.33-3.35 (m, 4H, 2×NCH₂CH₂), 6.56 (m, 2H, 3,5-Ph), 6.85 (d, ³*J* = 16.1 Hz, 1H, CH=CH), 7.18 (t, *J* = 7.0 Hz, 1H, 5-H_{Ind}), 7.30 (d, *J* = 7.0 Hz, 1H, 7-H_{Ind}), 7.31 (t, *J* = 7.0 Hz, 1H, 6-H_{Ind}), 7.50-7.51 (m, 2H, 2,6-Ph), 7.59 (d, *J* = 7.0 Hz, 1H, 4-H_{Ind}), 7.69 (d, ³*J* = 16.1 Hz, 1H, CH=CH). ¹³C NMR (175 MHz. CDCl₃): δ 24.3, 25.6, 47.7, 52.6, 111.9, 114.2, 120.1, 121.1 123.4, 124.9, 127.8, 129.3, 139.0, 146.6, 148.8, 154.5, 184.3. FT-IR (cm⁻¹): 3031, 3042 C-H_{arom.}; 2846, 2927, 2961 C-H_{alif.}; 1597 (CH=CH). HRMS (ESI) m/z [M + H]⁺ calculated C₂₂H₂₅N₂ 317.2018; found 317.2012.

The synthesis of compound **Ig** was also attempted in a flask. Initial 3*H*-indole hydrochloride (1 mmol, 1 equiv.) and 4-(1-pyrrolidinyl)benzaldehyde (1.3 equiv.) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 1:2, v/v). The yield of product **Ig** was 72%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

### Example 9

### (E)-3,3-dimethyl-2-[2-(naphthalen-1-yl)ethen-1-yl]-3H-indole (Ih);

Reaction according to the general flow synthesis description gave 196 mg (66%) of compound **Ih** from 2,3,3-trimethyl-3*H*-indole hydrochloride (195 mg, 1 mmol) and 1-naphthaldehyde (0.15 mL, 1.1 mmol). A yellow/orange amorphous material was obtained. ¹H NMR (700 MHz, CDCI₃): δ 1.53 (s, 6H), 7.16 (d, *J*= 16.1 Hz, 1H), 7.23 - 7.29 (m, 1H), 7.36 (dd, *J* = 14.6, 8.1 Hz, 2H), 7.50 - 7.56 (m, 2H), 7.58 (t, *J* = 7.6 Hz, 1H), 7.68 (d, *J =* 7.6 Hz, 1H), 7.85 - 7.91 (m, 3H), 8.29 (d, *J* = 8.4 Hz, 1H), 8.54 (d, *J =* 16.1 Hz, 1H).¹³C NMR (175 MHz, CDCl₃): δ 24.0, 52.8, 120.8, 121.1, 122.7, 123.6, 124.4, 125.6, 125.7, 126.1, 126.6, 127.9, 128.7, 129.6, 131.4, 133.6, 133.7, 134.9, 146.6, 153.9, 183.3. FT-IR (cm⁻¹): 3057, 3046 C-Hₐᵣₒₘ, 2963, 2926, 2864 C-H_{alif}, 1712, 1614, 1514, 1452, 1354, 1208, 968, 795, 772, 752. HRMS (ESI) m/z [M + H]⁺ calculated C₂₂H₂₀N 298.1590; found 298.1592.

The synthesis of compound **Ih** was also attempted in a flask. Initial 3*H*-indole hydrochloride (1 mmol, 1 equiv) and 1-naphthaldehyde (1.3 equiv) were dissolved in ethylene glycol (10 mL). The reaction mixture was heated in a flask with backflow cooler at 125 °C for 1 h. The cooled reaction mixture was transferred to a separation funnel, diluted and extracted with ethyl acetate (20 mL). The organic solution was washed with water (3 × 20 mL) followed by brine. The organic layer was dried over Na₂SO₄ and filtered. The solvent was removed using a rotary evaporator. The reaction product was purified by column chromatography (stationary phase - silica gel, eluent hexane/ethyl acetate, 10:1, v/v). The yield of product **Ih** was 33%.

Unlike synthesis in a flask, the flow reactor gave a better yield of the product. In addition, it was observed that the reaction in the flask required longer heating and excess aldehyde, which increased the formation of by-products. The formation of certain side compounds in the flask during reactions made it difficult to purify the product by chromatography due to the close R_{f} values. Thus, even with chromatographic purification, the yield of the reaction in the flask was lower and the purity of the product was lower.

The yields of compounds **Ia-Ih** for reactions according to **examples 2-8** in a flow reactor and in a flask are presented in Table 1

**Table 1**

| Compoun d number | Compound structure | Yield (in flow reactor), % | Yield (in flask), % |
|---|---|---|---|
| **Ia** | 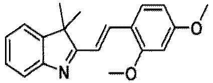 | 76 | 65 |
| **Ib** | 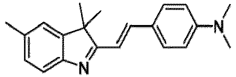 | 93 | 81 |
| **Ic** | 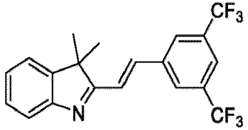 | 52 | 15 |
| **Id** | 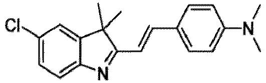 | 93 | 80 |
| **Ie** | 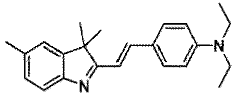 | 95 | 82 |
| **If** | 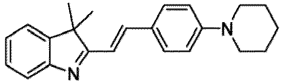 | 69 | 55 |
| **Ig** | 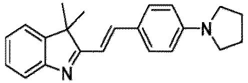 | 85 | 72 |
| **Ih** | 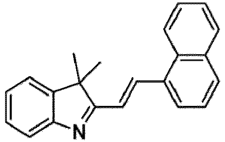 | 66 | 33 |

For comparison, known compounds (including CN102942515, A) were also synthesized using the proposed method in a flow reactor (60 min., 125 °C, solvent - ethylene glycol):
(*E*)-3,3-dimethyl-2-(4-methoxystyryl)-3*H*-indole (84% yield obtained compared to 57.2%);
(*E*)-3,3-dimethyl-2-(3,4-dimethoxystyryl)-3*H*-indole (82% yield obtained compared to 51.5%);
(*E*)-3,3-dimethyl-2-(4-dimethylaminostyryl)-3*H*-indole (94% yield obtained compared to 78.4%);
(*E*)-2-(4-diethylaminostyryl)-3,3-dimethyl-3*H*-indole (90% yield obtained compared to 69.8%).

That is, under the conditions of the proposed method, not only an obvious increase in the speed of the process is achieved (from 4 hours in the flask to 60 min), but also a significant increase in yield. This is related to the reaction time, where the reaction at a higher temperature for a shorter time reduces the formation of by-products, and at the same time makes it easier to purify the target product.

It turned out that the new compounds are characterized by the fact that they change color depending on the acidity of the medium from yellow-orange to red-violet (compounds **Ia, b, d-g**) or from yellow to orange (compound **Ih**) in the visible spectrum.

**Table 2**

| No . | Structural formula | pH | λₘₐₓ, nm EtOH/H₂O, 1:1 (v/v) | ε, l/mol·cm | pH | λₘₐₓ, nm EtOH/H₂O 1:1 (v/v) | ε, l/mol·cm |
|---|---|---|---|---|---|---|---|
| **Ia** | 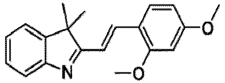 | 6.1 | 363 | 8968 | 1.5 | 453 | 22424 |
| **Ib** | 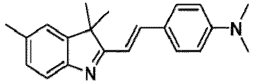 | 6.3 | 410 | 24801 | 1.7 | 542 | 51272 |
| **Id** | 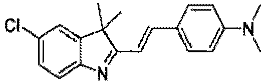 | 6.1 | 363 | 91267 | 1.7 | 555 | 74251 |
| **Ie** | 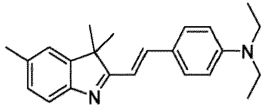 | 6.3 | 420 | 59287 | 1.6 | 549 | 85612 |
| **If** | 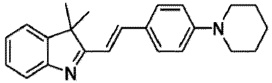 | 5.9 | 386 | 16605 | 1.5 | 547 | 9378 |
| **Ig** | 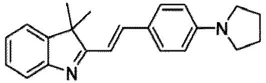 | 5.9 | 420 | 55307 | 1.7 | 551 | 51735 |
| **Ih** | 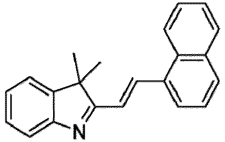 | 3.7 | 355 | 9476 | 2.3 | 431 | 8793 |

From the data presented in Table 2 it can be concluded that the compounds of the invention can be effective as color sensors sensitive to changes in pH. The compounds according to the invention can be used as pH indicators, which are reddish purple at pH < 1.5÷1.7, and yellow-orange at pH > 5.9÷6.3 (compounds **Ia, b, d-g**), or orange at pH < 2.3, and yellow at pH > 3.7 (compound **Ih**).

That is, the present invention has the following main advantages over the prior art:
1. The condensation reaction of 3*H*-Indole chlorides in the proposed method takes place several times faster (e.g., 60 min) than in a flask (3-12 h).
2. Good temperature control in a flow reactor process results in fewer by-products, easier compound purification and higher yields than in a flask.
3. By conducting condensation in the proposed method in a flow reactor, the process is more sustainable, because the solvent - ethylene glycol is used, part of the solvent (ethylene glycol) is returned to the production cycle, and also, since there is no extraction, no water is used.
4. The obtained new 3*H*-indole derivatives with a simple structure expand the variety of useful compounds that can be applied to detect pH changes in the medium.
5. Using a flow reactor, the entire class of 3,3-dimethyl-2-styryl-3*H*-indoles can be synthesized at lower cost, as the energy consumption for product production is reduced due to the reduction in reaction time; and due to reduced by-product formation, the product is easier to clean.
6. Water consumption is reduced because there is no extraction process and the solvent used in the reaction is returned to the synthesis cycle.
7. 3,3-Dimethyl-2-styryl-3*H*-indoles can be used as pH sensors, or intermediate products for the synthesis of other special dyes (pH sensors of biological media, cell markers, etc.).

The compounds by the patent can be used directly as pH indicators or they can be used as building blocks to create sophisticated pH indicators for monitoring pH changes in various biological systems and processes. The compounds described in the patent can be used in the chemical industry, medical diagnostics, etc. The flow synthesis method described in the patent makes it possible to synthesize organic compounds, 3,3-dimethyl-2-styryl-3*H*-indoles, in the chemical industry, without using water in the production process, thus making the manufacturing process closer to green.

## Claims

1. Method for obtaining 3,3-dimethyl-2-styryl-3*H*-indole derivatives according to formula I 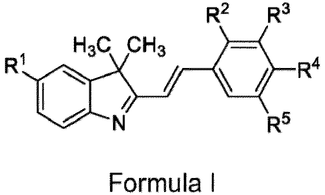 wherein
R¹ represents hydrogen, halogen, alkyl or aryl radical;
R² represents hydrogen or methoxy;
R³ represents hydrogen, methoxy or trifluoromethoxy; or
R² and R³ together are a chain from -CH=CH-CH=CH-;
R⁴ represents a hydroxy, methoxy, dimethylamino, diethylamino, piperidin-1-yl-, pyrrolidin-1-yl- or morpholin-4-yl- radical;
R⁵ represents hydrogen or trifluoromethoxy radical.
the production method comprises condensing 3,3-dimethyl-3*H*-indole salt with the corresponding aromatic aldehyde in ethylene glycol,
**characterized in that** the reaction is carried out in a flow reactor and comprises the following steps:
a) preparing solutions of the initial compounds, 2,3,3-trimethyl-3*H*-indole salt (A) and the corresponding aromatic aldehyde (B) in ethylene glycol in separate reservoirs;
b) feeding the prepared solutions (A) and (B) into the T-shaped mixer maintaining a constant flow;
c) after mixing, the reaction mixture, moving at the given speed, is heated in the tubular zone of the flow reactor, optimally for 50-70 min, preferably for 60 min, optimally at 120-130 °C, preferably at 125 °C;
d) cooling the continuous flow of the solution containing the reaction product in the reactor loop with ambient air, optimally to about 30 °C;
e) carrying out separation of unreacted aldehyde;
f) followed by separation of the ethylene glycol;
g) obtained reaction product is purified in a chromatographic column.

2. A method according to claim 1, **characterized in that:**
- before stage a), the flow reactor is filled with solvent, i.e. ethylene glycol, and heated to the reaction temperature;
- the initial compounds of step a) are 2,3,3-trimethyl-3*H*-indole hydrochloride (A) and the corresponding substituted benzaldehyde (B);
- the solutions A and B prepared in stage b) are supplied independently but simultaneously in a molar ratio of 1:1.1, mixed in a T-mixer and fed to the reactor at a given flow rate, for example 0.266 mL/min
- in stage d) the reaction is cooled
- to separate the unreacted aldehyde in stage e), the reaction flow is passed through a chromatographic column with an isoniazid-modified sorbent whose sorption capacity for aldehyde is 0.11-0.28 mmol/g;
- separation of the solvent in stage f) is carried out by evaporating ethylene glycol in a vacuum evaporator and returning the separated ethylene glycol to the stage a) for the preparation of initial solutions.

3. Compounds obtained by method according to any of the previous claims, wherein
R¹ represents hydrogen, Cl or methyl;
R² represents hydrogen or methoxy;
R³ represents hydrogen;
R³ represents trifluoromethyl when R⁵ represents trifluoromethyl; or
R² and R³ together are a chain from -CH=CH-CH=CH-;
R⁴ represents hydrogen, piperidin-1-yl-, pyrrolidin-1-yl-;
R⁴ represents methoxy when R² represents methoxy;
R⁴ represents dimethylamino, diethylamino, when R¹ does not represent a hydrogen radical;
R⁵ represents hydrogen.

4. 3,3-dimethyl-2-styryl-3*H*-indole derivatives according to claim 3, which are selected from the following compounds:
(E)-3,3-dimethyl-2-(2,4-dimethoxystyryl)-3*H*-indole (la);
(*E*)-3,3,5-trimethyl-2-(4-dimethylaminostyryl)-3*H*-indole (Ib)
(*E*)-3,3-dimethyl-2-(3,5-ditrifluoromethylstyryl)-3*H*-indole (Ic)
(*E*)-5-chloro-3,3-dimethyl-2-(4-dimethylaminostyryl)-3*H*-indole (Id)
(*E*)-2-(4-diethylaminostyryl)-3,3,5-trimethyl-3*H*-indole (Ie)
(*E*)-3,3-dimethyl-2-[4-(piperidin-1-yl)styryl]-3*H*-indole (If);
(*E*)-3,3-dimethyl-2-[4-(pyrrolidin-1-yl)styryl]-3*H*-indole (Ig); or
(*E*)-3,3-dimethyl-2-[2-(naphthalen-1-yl)styryl]-3*H*-indole (Ih);

5. Use of the 3,3-dimethyl-2-styryl-3*H*-indole derivatives according to one of claims 3-4, as a pH change indicator in the range from 1.3÷1.7 to 5.9 ÷6.3.
